# EUROPEAN PATENT APPLICATION

(11) **EP 4 692 326 A1**
(43) Date of publication of application: **11.02.2026**
(21) Application number: 24778078.6
(22) Date of filing: 27.03.2024
(51) Int. Cl.: C12N 5/0783, A61K 35/17, A61P 31/00, A61P 35/00, A61P 37/02

(54) **METHOD FOR INDUCING AND AMPLIFYING STEM MEMORY T CELL IN VITRO**

(30) Priority: 28.03.2023 CN 202310314122
(71) Applicant: Suzhou Institute Of Systems Medicine, Suzhou, Jiangsu 215123 (CN)
(72) Inventor: LI, Guideng, Suzhou, Jiangsu 215123 (CN); QIU, Yajing, Suzhou, Jiangsu 215123 (CN); CHENG, Hongcheng, Suzhou, Jiangsu 215123 (CN); DU, Jing, Suzhou, Jiangsu 215123 (CN); XIE, Ermei, Suzhou, Jiangsu 215123 (CN)
(74) Representative: ZBM Patents - Zea, Barlocci & Markvardsen
(86) International application number: PCT/CN2024/084144
(87) International publication number: WO 2024/199292

(57) **Abstract**

A method for inducing and expanding a stem memory T cell *in vitro.* The method includes obtaining a large number of stem memory T cells by means of changing the culture condition of T cells. The prepared stem memory T cell has a multidirectional differentiation potential and is suitable for any clinical adoptive immunotherapy, including tumor and infection immunity, autoimmune diseases, etc.

## Description

### TECHNICAL FIELD

The present disclosure relates to the field of bioengineering, and particularly to a method for inducing and expanding a stem memory T cell *in vitro.*

### BACKGROUND

Following surgery, radiotherapy and chemotherapy, T cell immunotherapy has become a new type of treatment for tumors, which is a method of triggering an immune response in the body by infusing ex vivo activated and expanded T cells of allogeneic or autologous origin to achieve the purpose of treating or alleviating diseases, and is considered to be a highly promising strategy for tumor treatment currently. However, this method is mostly based on terminal effector T cells currently, which are difficult to survive in the body for a long period of time, greatly affecting the clinical efficacy of adoptive cell therapy. It has been shown in recent studies that less differentiated T cells have stronger anti-tumor effects, especially the recently reported stem memory T cells (T_{SCM}) which, compared with terminal effector T cells, have stronger expansion, self-renewal and survival capabilities and can differentiate into central memory T cells and effector memory T cells. In a mouse anti-tumor model, it was found that adoptive stem memory T cells exhibited stronger anti-tumor effects than adoptive central memory T cells or effector memory T cells.

Stem memory T cells are the most ideal group of cell subpopulations for adoptive T cell therapy, but since it is difficult for them to expand under conventional culture conditions, there is still no effective method to rapidly induce stem memory T cells *in vitro* so far. It has been reported that after transient effect of CD3/CD28 co-stimulatory molecules, the combined use of cytokines IL-7, IL-15 and IL-21 can improve the induction ratio of stem memory T cells and culture a larger number of stem memory T cells, thereby providing a more optimized culture regimen for adoptive T cell therapy. However, during the large-scale induction of stem memory T cells, cytokine drugs are used in large quantity, greatly increasing the production cost. At the same time, flow cytometer sorting technology is used to obtain a large number of stem memory T cells, but this technology is not suitable for large-scale cell production due to the high requirements on the instrument and operating environment, and there is a high risk of microbial infection in the process. Therefore, it becomes particularly important to explore a rapid method for preparing stem memory T cells with high efficiency, low cost, and a high safety factor.

Some studies have shown that appropriate inhibition of T cell metabolism during the *in vitro* expansion of T cells can maintain T cells in a low-differentiated state. For example, rapamycin can keep T cells in a stemness state by inhibiting their glycolysis process. Mannose is an isomer of glucose, distributed in human blood and various tissues, such as skin, organs, etc. Mannose can participate the synthesis of glycoprotein and mediate the function of the autoimmune system. At the same time, mannose can inhibit the tumor growth by interfering with glucose metabolism and PD-L1 protein stability in tumor cells.

### SUMMARY

### Problem to be Solved

A technical problem that urgently needs to be solved by those skilled in the art is how to provide a method for rapidly and efficiently inducing and expanding a stem memory T cell *in vitro.*

### Technical Solution for Solving the Problem

In one aspect, the present disclosure provides a method for inducing and expanding a stem memory T cell *in vitro,* where the method includes the following steps:
S1: activating and expanding a T cell;
S2: adding mannose to a culture medium to culture the T cell during or after the activation of the T cell.

Preferably, the step S1 includes: coating a cell culture plate with an antibody, then activating and expanding the T cell in a culture medium containing a cytokine.

Preferably, the antibody is a CD3 antibody and a CD28 antibody.

Preferably, the cytokine is selected from one or more of a group consisting of IL-2, IL-7, IL-15, IL-17, and IL-21.

Preferably, the cytokine is IL-2.

Preferably, a concentration of the CD3 antibody is 1-2 µg/mL.

Preferably, a concentration of the CD28 antibody is 1-2 µg/mL.

Preferably, a concentration of the mannose is 2.0-100.0 mM.

Preferably, the concentration of the mannose is 5.0-50.0 mM.

Preferably, the method further includes the following step:
S3: replacing the culture medium with a culture medium containing a cytokine and mannose for culture to obtain a lymphocyte population primarily composed of the stem memory T cell.

Preferably, the cytokine is selected from a group consisting of one or more of IL-2, IL-7, IL-15, IL-17, and IL-21.

Preferably, the cytokine is IL-2.

Preferably, time for replacing the culture medium is 1 to 3 days.

Preferably, the time for replacing the culture medium is 2 days.

Preferably, the lymphocyte population includes CD4 T cells, CD8 T cells, γδ T, TILs, TCR-T, CAR-T, and STAR-T.

Preferably, the method further includes the following step:
S4: stimulating the T cell obtained in the step S3 with an antibody for further culture.

Preferably, the culture is conducted for 12 days.

Preferably, the step S4 is conducted cyclically for two or more times.

Preferably, the antibody is a CD3 antibody and/or a CD28 antibody.

Preferably, the antibody is the CD3 antibody.

Preferably, a concentration of the antibody is 0.5-2 µg/mL.

Preferably, the concentration of the antibody is 1 µg/mL.

Preferably, the T cell includes a human-derived T cell or a murine-derived T cell.

In another aspect, the present disclosure provides a lymphocyte population prepared by the method above.

In another aspect, the present disclosure provides a pharmaceutical composition including the lymphocyte population above.

Preferably, the pharmaceutical composition further includes a pharmaceutically acceptable excipient.

Preferably, the pharmaceutical composition further includes an additional therapeutic agent.

In another aspect, the present disclosure provides a use of the lymphocyte population prepared by the method above, the lymphocyte population above or the pharmaceutical composition above in the preparation of a drug for adoptive immunotherapy.

Preferably, the adoptive immunotherapy is anti-tumor immunotherapy, anti-infection immunotherapy, and autoimmune disease treatment.

In another aspect, the present disclosure provides a use of mannose in inducing and expanding a stem memory T cell *in vitro.*

### Technical Effects

1. The compounds used in the present disclosure are relatively simple and cheap, having characteristics of low price and high production safety. A large number of stem memory T cells can be obtained by means of changing the culture condition of T cells, reducing the use of other cytokines and greatly decreasing the toxic side effects generated from the use of cytokines, thus reducing the production cost of stem memory T cells.
2. The stem memory T cells obtained by the present disclosure have a multi-directional differentiation potential, and the differentiated T cells can be efficiently recruited to the site where they exert their functions to capture and eliminate antigens or tumor cells or pathogens carrying the antigens, while maintaining their stem cell-like T cell properties *in vivo,* thereby creating conditions for further differentiation into effector T cells, thus extending the *in vivo* persistence of adoptive T cells in adoptive immunotherapy and producing long-term protective efficacy.
3. The stem memory T cells obtained from the present disclosure are suitable for any clinical adoptive immunotherapy, including, but not limited to, tumor and infection immunity, autoimmune diseases, etc.

### BRIEF DESCRIPTION OF THE DRAWINGS

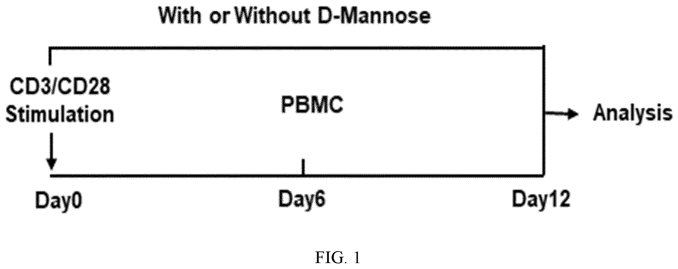

FIG. 1 is a schematic diagram showing the induction and expansion of stem memory T cells in PBMC.

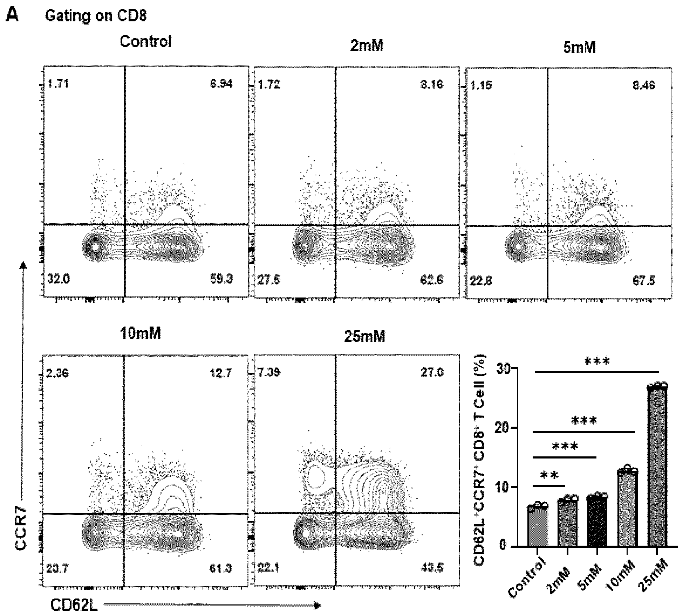

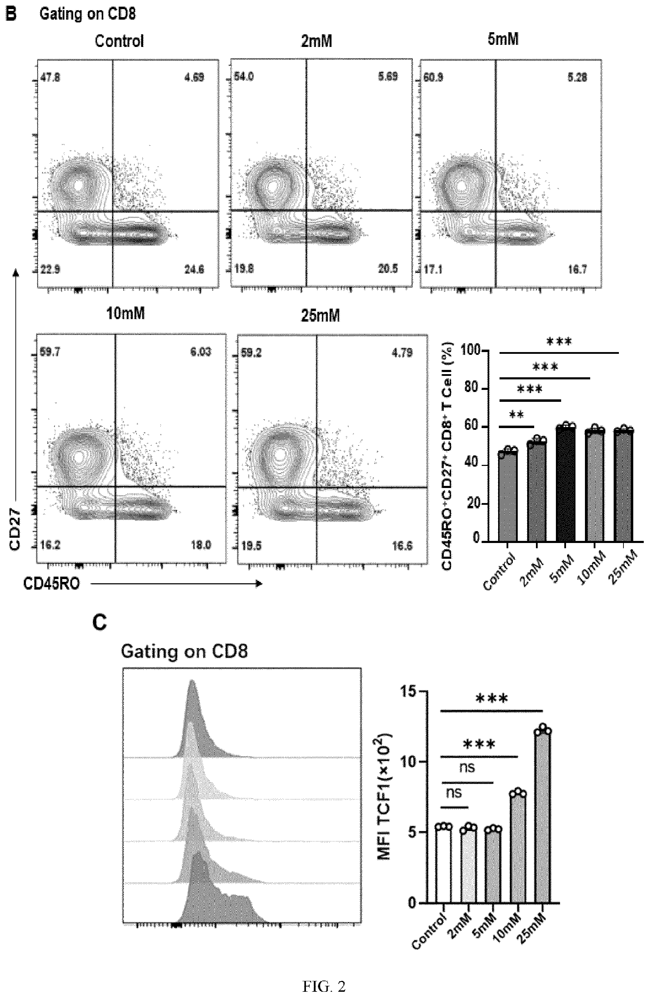

FIG. 2 shows that treatment with mannose significantly promotes the formation of stem memory T cells in PBMCs; where, A and B show the proportion of CD62L⁺CCR7⁺ (T_{SCM}) and CD45RO⁻CD27⁺ (T_{SCM}) T cells when gating on CD8, respectively; C shows the mean fluorescence intensity of T cell stem transcription factor TCF-1 when gating on CD8.

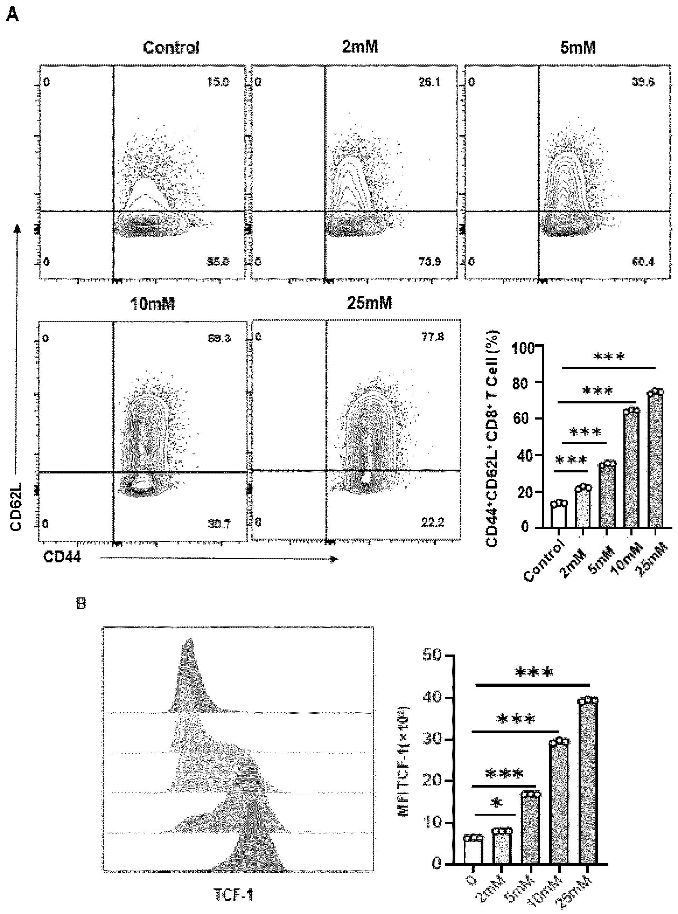

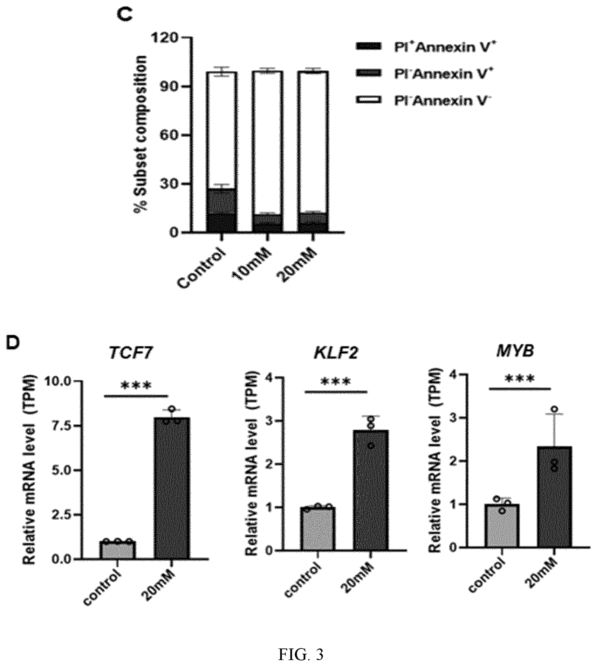

FIG. 3 shows that treatment with mannose significantly promotes the formation of murine stem memory T cells; where, A shows the proportion of CD44⁺CD62L⁺ (T_{CM}) T cells when gating on CD8; B shows the mean fluorescence intensity of T cell stem transcription factor TCF-1 when gating on CD8; C shows the effect of mannose on the proportion of T cell apoptosis; D shows the expression level of T cell stem-related transcription factor in the transcriptome sequencing results of T cells treated with mannose (20mM).

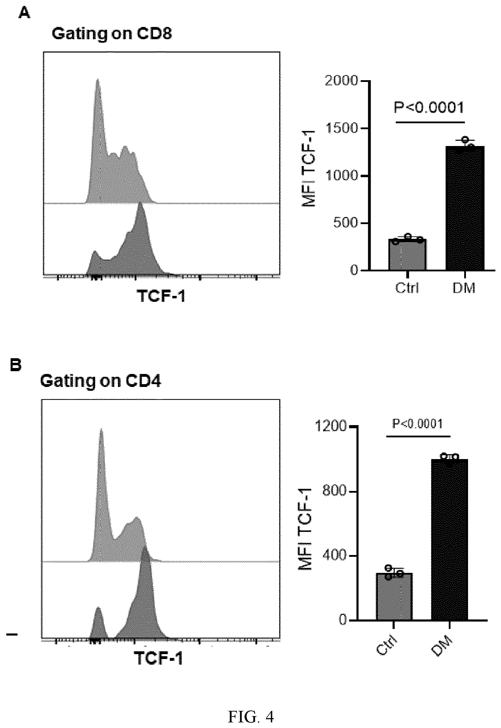

FIG. 4 shows that treatment with mannose (DM, 50mM) significantly promotes the formation of stem memory T cells in 1G4 TCR T cells; where, A shows the mean fluorescence intensity of T cell stem transcription factor TCF-1 when gating on CD8; B shows the mean fluorescence intensity of T cell stem transcription factor TCF-1 when gating on CD4.

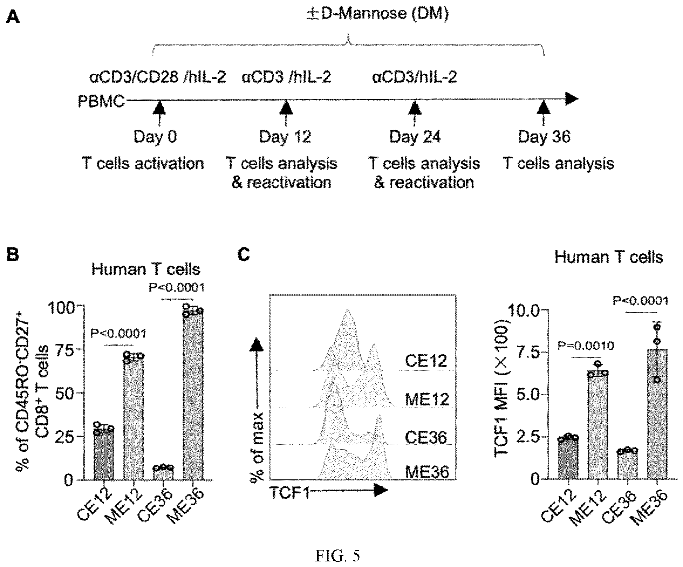

FIG. 5 shows that long-term *in vitro* treatment with mannose (DM, 50mM) significantly increases the formation of stem memory T cells; where, A is the experimental flow chart of long-term treatment of T cells with mannose; B shows the proportion of CD45RO⁻CD27⁺ stem memory T cells when gating on CD8; C shows the mean fluorescence intensity of T cell stem transcription factor TCF-1 when gating on CD8.

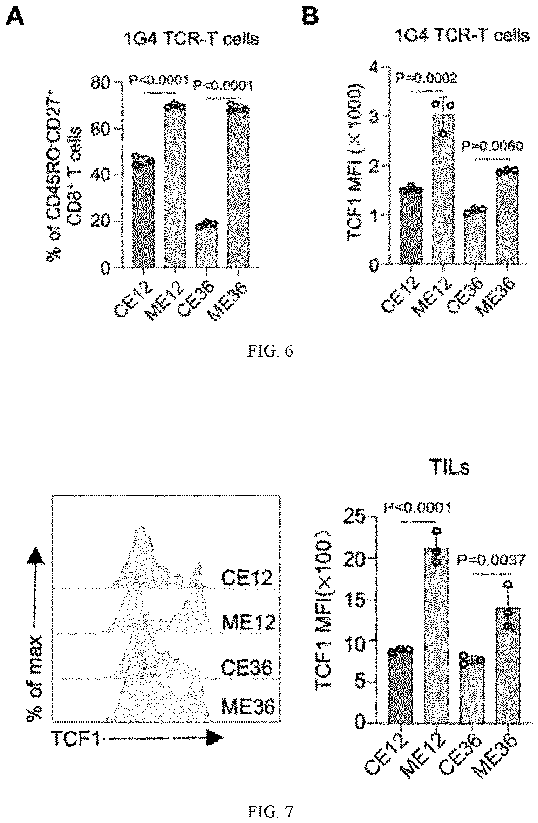

FIG. 6 shows that long-term *in vitro* treatment with mannose (DM, 50mM) significantly increases the proportion of 1G4 TCRT cells; where, A shows the proportion of CD45RO⁻CD27⁺ stem memory T cells when gating on CD8; B shows the mean fluorescence intensity of T cell stem transcription factor TCF-1 when gating on CD8.
FIG. 7 shows that long-term *in vitro* treatment with mannose (DM, 50mM) significantly promotes the formation of stem memory T cells in TIL cells; the figure shows the mean fluorescence intensity of T cell stem transcription factor TCF-1 when gating on CD8.

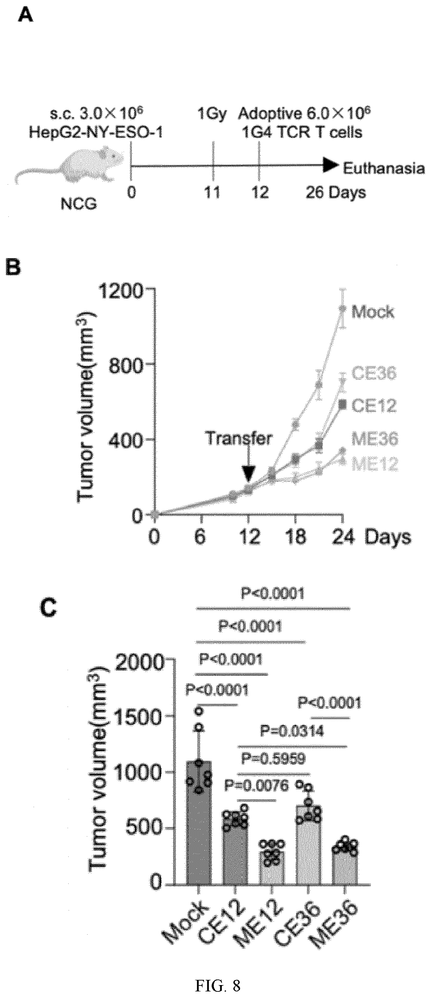

FIG. 8 shows that T cells (CE12/ME12/CE36/ME36) treated with mannose (DM, 50mM) for a prolonged period of time exhibit strong anti-tumor activity *in vivo;* where, A is a schematic diagram of *in vivo* experiment of HepG2-NYESO tumor-bearing mice; B and C show the growth curves and tumor volume statistics of HepG2-NYESO tumors after adoptive transfer of 1G4 TCR T cells from different treatment groups (CE12/ME12/CE36/ME36), respectively.

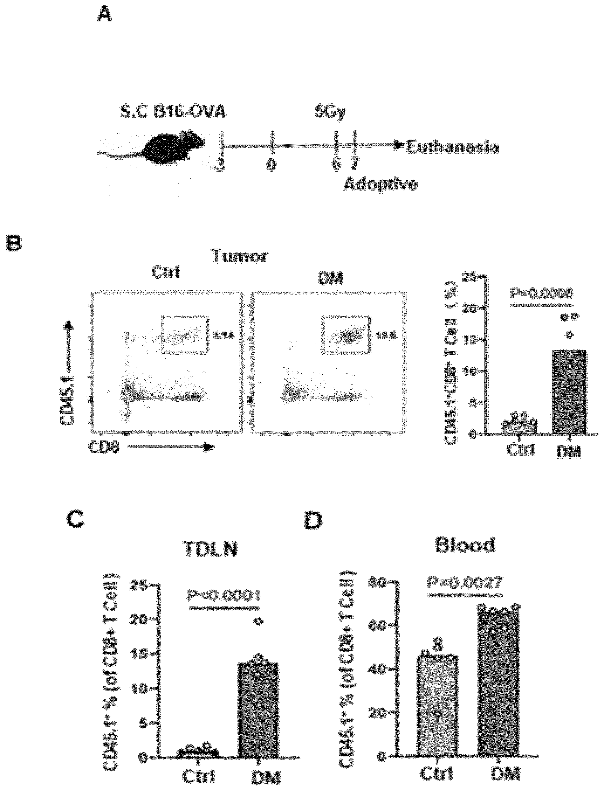

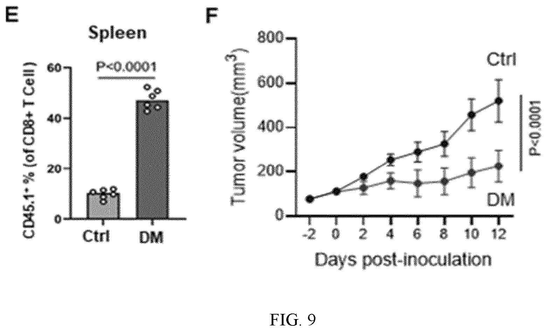

FIG. 9 shows that T cells treated with mannose (DM, 20mM) exhibit strong *in vivo* anti-tumor activity and persistence; where, A is a schematic diagram of *in vivo* experiment of B16-OVA tumor-bearing mice; B, C, D, and E show the proportion of adoptive T cells in tumors, tumor-draining lymph nodes (TDLNs), blood and spleen of mice after adoptive transfer of OT-I T cells from different treatment groups (control group and mannose treatment group), respectively; F shows the growth curve of B16-OVA tumor after adoptive transfer of OT-I T cells from different treatment groups (control group and mannose treatment group).

### DESCRIPTION OF THE EMBODIMENTS

In order to make the technical solutions and beneficial effects of the present disclosure more apparent and easier to understand, the following detailed description is provided through specific embodiments. The accompanying drawings are not necessarily drawn to scale, and localized features may be enlarged or reduced to show details of localized features more clearly. Unless otherwise defined, the technical and scientific terms used herein have the same meanings as those in the technical field to which the present disclosure belongs.

In one aspect, the present disclosure provides a method for inducing and expanding a stem memory T cell *in vitro,* where the method includes the following steps:
S1: activating and expanding a T cell;
S2: adding mannose to a culture medium to culture the T cell during or after the activation of the T cell.

In some embodiments, the step S1 includes: coating a cell culture plate with an antibody, then activating and expanding the T cell in a culture medium containing a cytokine.

In some embodiments, the antibody is a CD3 antibody and a CD28 antibody.

In some embodiments, the cytokine is selected from one or more of a group consisting of IL-2, IL-7, IL-15, IL-17, and IL-21.

In some embodiments, the cytokine is selected from IL-2.

In some embodiments, a concentration of the CD3 antibody is 1 to 2 µg/mL.

In some embodiments, the concentration of the CD3 antibody is about 1 µg/mL.

In some embodiments, the concentration of the CD3 antibody is about 1.5 µg/mL.

In some embodiments, the concentration of the CD3 antibody is about 2 µg/mL.

In some embodiments, a concentration of the CD28 antibody is 1 to 2 µg/mL.

In some embodiments, the concentration of the CD28 antibody is about 1 µg/mL.

In some embodiments, the concentration of the CD28 antibody is about 1.5 µg/mL.

In some embodiments, the concentration of the CD28 antibody is about 2 µg/mL.

In some embodiments, a concentration of the mannose is 2.0-100.0 mM.

In some embodiments, the concentration of the mannose is 5.0-50.0 mM.

In some embodiments, the concentration of the mannose is about 5.0mM, about 6.0mM, about 7.0mM, about 8.0mM, about 9.0mM, about 10.0mM, about 11.0mM, about 12.0mM, about 13.0mM, about 14.0mM, about 15.0mM, about 16.0mM, about 17.0mM, about 18.0mM, about 19.0mM, about 20.0mM, about 21.0mM, about 22.0mM, about 23.0mM, about 24.0mM, about 25.0mM, about 26.0mM, about 27.0mM, about 28.0mM, about 29.0mM, about 30.0mM, about 31.0mM, about 32.0mM, about 33.0mM, about 34.0mM, about 35.0mM, about 36.0mM, about 37.0mM, about 38.0mM, about 39.0mM, about 40.0mM, about 41.0mM, about 42.0mM, about 43.0mM, about 44.0mM, about 45.0mM, about 46.0mM, about 47.0mM, about 48.0mM, about 49.0mM, or about 50.0mM.

In some embodiments, the method further includes the following step:
S3: replacing the culture medium with a culture medium containing a cytokine and mannose for culture to obtain a lymphocyte population primarily composed of the stem memory T cell.

In some embodiments, the cytokine is selected from one or more of a group consisting of IL-2, IL-7, IL-15, IL-17, and IL-21.

In some embodiments, the cytokine is selected from IL-2.

In some embodiments, time for replacing the culture medium is 1 to 3 days.

In some embodiments, the time for replacing the culture medium is 2 days.

In some embodiments, the lymphocyte population includes CD4 T cells, CD8 T cells, γδ T, TILs, TCR-T, CAR-T, and STAR-T.

In some embodiments, the method further includes the following step:
S4: stimulating the T cell obtained in the step S3 with an antibody for further culture.

In some embodiments, the culture is conducted for 12 days.

In some embodiments, the step S4 is cyclically conducted for two or more times.

In some embodiments, the antibody is a CD3 antibody and/or a CD28 antibody.

In some embodiments, the antibody is a CD3 antibody and a CD28 antibody.

In some embodiments, the antibody is a CD3 antibody.

In some embodiments, the antibody is a CD28 antibody.

In some embodiments, a concentration of the antibody is 0.5-2 µg/mL.

In some embodiments, the concentration of the antibody is 0.5 µg/mL, or 0.6 µg/mL, or 0.7 µg/mL, or 0.8 µg/mL, or 0.9 µg/mL, or 1 µg/mL, or 1.1 µg/mL, or 1.2 µg/mL, or 1.3 µg/mL, or 1.4 µg/mL, or 1.5 µg/mL, or 1.6 µg/mL, or 1.7 µg/mL, or 1.8 µg/mL, or 1.9 µg/mL, or 2 µg/mL.

In some embodiments, the concentration of the antibody is 1 µg/mL.

In some embodiments, the T cell includes a human-derived T cell or a murine-derived T cell.

In some embodiments, the present disclosure provides a method for expanding a stem memory T cell *in vitro,* which includes the following step: isolating with dextran-meglumine diatrizoate mixture to obtain human peripheral blood mononuclear cells (PBMCs). Then inoculating the obtained PBMC in a 24-well culture plate coated with a human CD3 antibody, adding a free human CD28 antibody and a cytokine (such as rhIL-2, etc.) into the 24-well culture plate for activation and culture. Adding mannose in the culture medium at any time during the PBMC cells activation or within 3 days before activation, and culturing the cells under environment of 37°C, 5% CO₂, and saturated humidity. Replacing half of the culture medium and supplementing the culture medium with corresponding rhIL-2 after 3 days of T cells activation. After then, adding an equal amount of culture medium containing rhIL-2 and mannose every two days according to the growth status of the T cells to maintain the density of T cells in a range of 0.5-2×10⁶/ml. Obtaining the stem memory T cells after 9-15 days of culture. Continually stimulating the T cells cultured to day 12 with human CD3 antibody for 24 hours, transferring the stimulated T cells to a new cell culture plate, and continually culturing to day 24. Continually stimulating the T cells cultured to day 24 with human CD3 antibody for 24 hours, transferring the stimulated T cells to a new cell culture plate, and continually culturing the T cells to day 36.

In another aspect, the present disclosure provides a lymphocyte population prepared by the method above.

In another aspect, the present disclosure provides a pharmaceutical composition including the lymphocyte population above.

In some embodiments, the pharmaceutical composition further includes a pharmaceutically acceptable excipient.

In some embodiments, the pharmaceutical composition further includes an additional therapeutic agent.

In another aspect, the present disclosure provides a use of the lymphocyte population prepared by the method above, the lymphocyte population above or the pharmaceutical composition above in the preparation of a drug for adoptive immunotherapy.

In some embodiments, the adoptive immunotherapy is anti-tumor immunotherapy, anti-infection immunotherapy, and autoimmune disease treatment.

The present disclosure provides a method for treating an individual, which may include:
administering the lymphocyte population expanded as described above to an individual in need thereof.

The lymphocyte population can be administered to an individual intravenously, e.g., by means of infusion.

In another aspect, the present disclosure provides a use of mannose in inducing and expanding a stem memory T cell *in vitro.*

Unless otherwise defined in the specification, the technical and scientific terms used herein have the same meanings as commonly understood by those skilled in the art to which the present disclosure belongs by reference to the content of the disclosure.

The term "a" or "an" refers to one or more, for example, "a molecule" should be understood to indicate one or more molecules. Therefore, the term "a" or "an", "one or more", and "at least one" can be used interchangeably herein.

In the claims and specification of the present disclosure, unless otherwise required by the context due to expression language or necessary implication, the word "comprise" or variants thereof, such as "comprises" or "comprising", etc., are used in an inclusive sense, that is, the presence of the stated features does not exclude the presence or addition of other features in various embodiments of the present disclosure.

The term "about" encompasses values within a range of ±25% of the magnitude of the given value. In other embodiments, the term "about" encompasses values within a range of ±20%, ±15%, ±10% or ±5% of the magnitude of the given value. For example, "about 3 g" indicates values from 2.7 to 3.3 grams (i.e., 3 g ± 10%).

The term "T cell" refers to a cell with TCR or TCR genetically edited or modified on the cell surface. In the present specification, "T cell" may be a cell population including other cell types other than T cells themselves, e.g., a cell population including more than 60%, more than 70%, more than 80%, more than 90% or more than 95% of T cells. Additionally, the T cell includes T cells at all stages of differentiation, and may be a T cell that has an antigen or marker on its cell surface.

The term "stem memory T cell" and "memory stem cell-like T cell" generally refer to a T memory stem cell (T_{SCM}) at early differentiation stage of a memory T cell, which has the characteristics of stem cell and has strong multi-directional differentiation potential. T_{SCM} cells, in response to antigen stimulation, can differentiate into central memory T cells (T_{CM}), effector memory T cells (T_{EM}) and effector T cells (T_{EF}), thereby producing a large number of effectors such as IFN-γ, etc., generating more powerful killing ability, and thus having a strong self-renewal ability while having the differentiation potential.

The term "mannose" generally refers to a kind of carbohydrates. For example, the mannose may have CAS No. 3458-28-4. In the present disclosure, mannose may refer to any one derivative of mannose, and the mannose may be extracted naturally or synthesized artificially.

The term "culture medium" generally refers to a culture medium enabling the stem cells to grow normally. In some implementations, the stem cell culture medium also makes the stem cells in a suspension state. For example, in the present disclosure, the stem cell culture medium may include DMEM culture medium, 1640 culture medium, MEM culture medium, and X-VIVO culture medium.

The term "CD3 antibody" generally refers to an antigen-binding molecule specifically binding to CD3. For example, the CD3 antibody may include T3, I, eu4, and HCHT1. The CD3 molecule is connected to the T cell antigen receptor through a salt bridge and participates in the signal transduction of T cells.

The term "CD28 antibody" generally refers to an antigen-binding molecule specifically binding to CD28. Human CD28 is located at 2q33 and has similar exons and introns to CTLA4, the ligands of both being B7 family, including B7-1 (CD80) and B7-2 (CD86). CD28 and CD80 have high aggregate affinity and can interact with a variety of signaling molecules or kinases, such as PI-3K, GRB-2/s and tyrosine kinase ITK.

The term "cytokine" generally refers to a class of micromolecular protein with extensive biological activities synthesized and secreted upon the stimulation of immune cells (e.g., monocytes, macrophages, T cells, B cells, NK cells, etc.) and some nonimmune cells (endothelial cells, epidermal cells, fibroblasts, etc.). The cytokine may has various functions of regulating innate and adaptive immunity, hemocytogenesis, cell growth, APSC pluripotent cells, and damaged tissue repair, and the like. In the present disclosure, the cytokine may include an interleukin, an interferon, tumor necrosis factor superfamily, a colony stimulating factor, a chemokine, and a growth factor. For example, the cytokine is a interleukin.

The term "interleukin" generally refers to secretory protein or signaling molecules capable of promoting the development and differentiation of T and/or B lymphocytes and/or hematopoietic cells. The interleukin can be synthesized from helper CD4T lymphocytes as well as monocytes, macrophages, and endothelial cells. As used herein, the interleukin (IL) may be selected from a group consisting of IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, and IL-36. As used herein, the term "interleukin" may include full-length interleukin or fragments (e.g., truncated forms) or variants thereof, which substantially maintain the biological activity of a corresponding wild-type interleukin (for example, having at least 80%, at least 90%, at least 95%, at least 98%, at least 99%, or even at least 100% of the biological activity of the corresponding wild-type interleukin). The interleukin as used herein may derive from any mammal species. In some embodiments, the interleukin derives from species selected from a group consisting of human, horse, cow, mouse, pig, rabbit, cat, dog, rat, goat, sheep, and non-human primates. In some embodiments, the interleukin may be a mutated form. For example, the interleukin may be super-IL-2 (also known as sIL2, see Nature 484, 529-533, 2612), which can be obtained by modifying IL-2 to increase its binding affinity to IL-2Rβ. The mutations in sIL-2 are primarily located in the core of the cytokine. It is suggested by molecular dynamics simulations that evolutionary mutations stabilize IL-2 by reducing the flexibility of the helix in the IL-2Rβ binding site to a similarly optimized receptor binding conformation when bound to CD25. Compared to IL-2, sIL-2 induces excellent expansion of cytotoxic T cells, leading to improved anti-tumor response *in vivo,* and causing less expansion of T regulatory cells and reduced pulmonary edema. For example, in the present disclosure, the cytokine includes selected from one or more of a group consisting of I-L2, IL-7, IL-15, IL-17, and IL-21.

The term "pharmaceutical composition" refers to a lymphocyte population including the stem memory T cells described herein or primarily composed of the stem memory T cells that is formulated for administration to individuals. Preferably, the pharmaceutical composition is sterile. In one embodiment, the pharmaceutical composition is pyrogen-free.

The stem memory T cells or the lymphocyte population primarily composed of the stem memory T cells will be formulated, dosed and administered in accordance with good medical practice. Factors to be considered in this context include the type of specific disease being treated, the specific individual being treated, the individual's clinical condition, the site of administration, the method of administration, the scheduling of administration, possible side effects and other factors known to the physician.

The term "effective amount" refers to an amount of the stem memory T cells or the lymphocyte population primarily composed of stem memory T cells that is effective for treating a condition, disease or disorder in an individual.

The term "tumor" generally refers to physiological status characterized by unregulated cell growth in mammals. In the present disclosure, the tumor may include lymphoma, blastoma, sarcoma, and leukemia. For example, the tumor may include squamous-cell carcinoma, lung cancer (including small cell lung cancer, non-small cell lung cancer, lung adenocarcinoma, and lung squamous-cell carcinoma), peritoneal cancer, hepatocellular carcinoma, stomach cancer or gastric cancer (including gastrointestinal cancer), pancreatic cancer, glioblastoma, cervical cancer, ovarian cancer, liver cancer, bladder cancer, liver cancer, breast cancer, colon cancer, colorectal cancer, endometrial cancer or uterine cancer, salivary gland cancer, kidney or renal cancer, liver cancer, prostate cancer, vulvar cancer, thyroid cancer, liver cancer, head and neck cancer, B cell lymphoma (including low-grade/follicular non-Hodgkin lymphoma (NHL), small lymphocytic (SL) NHL, intermediate-grade/follicular NHL, intermediate-grade diffuse NHL, high-grade immunoblast NHL, high-grade lymphocytic NHL, high-grade small non-cleaved cell NHL, AIDS-related lymphoma, Waldenstrom's macroglobulinemia), chronic lymphocytic leukemia (CLL), acute lymphocytic leukemia (ALL), hairy cell leukemia, chronic myeloblastic leukemia, and post-transplant lymphoproliferative disorder (PTLD)).

The term "infection" disease generally refers to a disease caused by a virus invading the body through multiple pathways and replicating within susceptible host cells. For example, the viral infection diseases may include upper respiratory infection, bronchitis, viral pneumonia, viral enteritis, viral myocarditis, measles, chicken pox, rubella, exanthem subitum, hand-foot- and-mouth diseases, mumps, hepatitis B, severe acute respiratory syndrome (SARS), HIV, AIDS, avian influenza, and hepatitis A.

The term "autoimmune disease" generally refers to a disease caused by the immune response of the body against self-antigens resulting in damage to its own tissues. Autoimmune diseases can be divided into organ-specific autoimmune diseases and systemic autoimmune diseases. For example, the autoimmune diseases may include chronic lymphocytic thyroiditis, hyperthyroidism, insulin dependent diabetes mellitus, myasthenia gravis, chronic ulcerative colitis, pernicious anemia with chronic atrophic gastritis, goodpasture syndrome, pemphigus vulgaris, pemphigoid, primary biliary cirrhosis, multiple cerebrospinal sclerosis, acute idiopathic polyneuritis, systemic lupus erythematosus (SLE), rheumatoid arthritis, scleroderma, polyarteritis nodosa, and Wegener's granulomatosis.

The term "individual" refers to a mammal. The mammal may be a primate, especially a human, or may be a livestock, a zoo animal or a companion animal. In some preferred embodiments, the individual is a human. In other preferred embodiments, a non-human mammal can be used, especially a mammal (e.g., a murine, primate, swine, canine or leporid animal) that is conventionally used as a model for demonstrating the therapeutic effect in the human.

The method of the present disclosure will be illustrated below through the following specific embodiments. It should be understood that these embodiments are intended to explain the basic principles, main features, and advantages of the present disclosure, and the scope of the present disclosure is not limited by these embodiments. The implementation conditions used in the embodiments can be further adjusted according to specific requirements, and the implementation conditions not specified are generally those used in conventional experiments.

In the present disclosure, the PBMCs are obtained by means of, including but not limited to, density gradient centrifugation; the final concentration of rhIL-2 is in a range from 50 to 100 IU; the cytokine includes but not limited to IL-7, IL-15, IL-21, and any cytokines for *in vitro* T cell induction; the mannose is added within a concentration range from 2.0 to 100.0 mM, the recommended concentration range being from 10.0 to 50.0 mM.

### Embodiment 1 In vitro expansion method using human-derived stem memory T cells as primary cell population

This embodiment includes the following steps:
1. Coating a cell culture plate with human CD3 antibody at a final concentration of 1 µg/ml.
2. Collecting 10 ml of peripheral blood from healthy people in a centrifuge tube, diluting the peripheral blood with phosphate buffer solution (PBS) at a ratio of 1:1, and mixing the peripheral blood and the PBS evenly; adding 20 mL of lymphocyte separation medium (ficoll) into another new 50 mL centrifuge tube, then mixing the ficoll and blood dilution in a volume ratio of 1:1 to obtain the mixed blood dilution, slowly adding the mixed blood dilution along the tube wall to the upper layer of the lymphocyte separation medium to form a clear stratification between them, and centrifuging them at 800g for 20 min with an acceleration of 0 and a deceleration of 0.
3. At the end of the centrifugating operation, pipetting cells from the monocyte layer into a 50 mL centrifuge tube, washing the cells once by adding 20 mL of PBS, centrifuging them at 800g for 5 min, discarding the supernatant; then resuspending with T cell culture medium and counting the T cells, and diluting the cells to 1 million/ml to obtain T cell suspension.
4. Then adding human CD28 antibody at a final concentration of 1 µg/ml and 300 U/ml of rhIL-2 into the T cell suspension to mix them well, seeding the cells in the coated 24-well plate, adding a corresponding concentration of mannose, blowing the mannose to homogeneity and culturing the cells in an incubator. Then supplementing an equal amount of culture medium containing rhIL-2 and mannose every 2 days according to the growth status of T cells to maintain the density of T cells within a range of 0.5-2×10⁶/ml. On day 12 of culture, detecting the proportion of stem memory T cells and the expression level of transcription factors TCF-1 that regulate the differentiation of stem memory T cells in the culture by flow cytometry.
5. The results were shown in FIG. 2: on day 12 of the culture, the proportion of CCR7⁺CD62L⁺ and CD45RO⁻CD27⁺ stem memory T cells in the mannose treatment group was significantly higher than that of the control group under an ordinary culture condition, meanwhile the expression of transcription factors TCF-1 that regulate the stemness of T cells in the mannose-treated T cells was also significantly higher than that of the control group.

### Embodiment 2 In vitro expansion method using murine-derived stem memory T cells as primary cell population

This embodiment includes the following steps:
1. Coating a cell culture plate with murine CD3 antibody at a final concentration of 2 µg/ml.
2. Isolating mouse spleen cells, obtaining mouse T cells by sorting the mouse spleen cells with nylon hair column or magnetic beads, and diluting the cells to 1 million/ml with a T cell culture medium.
3. Adding murine CD28 antibody at a final concentration of 1 µg/ml and 20-50 U/ml of rmIL-2 into the T cells, adding a corresponding concentration of mannose, blowing the mannose to homogeneity, seeding the cells in the cell culture plate precoated with murine CD3 antibody, and culturing the cells in an incubator.
4. Then supplementing an equal amount of culture medium containing murine IL-2 recombinant protein and mannose every 24 hours to maintain the cell density within a range of 0.5-2×10⁶/ml. On day 6 of the culture of T cells, detecting the proportion of stem memory T cells in the culture and the expression level of transcription factors TCF-1 that regulate the differentiation of stem memory T cells by flow cytometry.
5. The results were shown in FIG. 3: on day 6 of the culture, the proportion of CD44⁺CD62L⁺ stem memory T cells in the mannose treatment group was increased from about 15.0% to about 77.8%, which was significantly higher than that of T cells under an ordinary culture condition; meanwhile the expression of transcription factors TCF-1 that regulate the stemness of T cells and the transcription level of T cell stem-related transcription factors in the mannose-treated T cells were significantly higher than those of the control group.

### Embodiment 3: In vitro expansion method of 1G4 TCR T cells using stem memory T cells as primary cell population

This embodiment includes the following steps:
1. Coating a cell culture plate with human CD3 antibody at a final concentration of 1 µg/ml.
2. Collecting 10 ml of peripheral blood from healthy people in a centrifuge tube, diluting the peripheral blood with phosphate buffer solution (PBS) at a ratio of 1:1, and mixing them evenly; adding 20 mL of lymphocyte separation medium (ficoll) into another new 50 mL centrifuge tube, then mixing the ficoll and blood dilution in a volume ratio of 1:1 to obtain the mixed blood dilution, slowly adding the mixed blood dilution along the tube wall to the upper layer of the lymphocyte separation medium to form a clear stratification between them, and centrifuging them at 800g for 20 min with an acceleration of 0 and a deceleration of 0.
3. At the end of the centrifugating operation, pipetting cells from the monocyte layer into a 50 mL centrifuge tube, washing the cells once by adding 20 mL of PBS, centrifuging them at 800 g for 5 min, discarding the supernatant; then resuspending with T cell culture medium and counting the T cells, and diluting the cells to 1 million/ml to obtain T cell suspension.
4. Then adding human CD28 antibody at a final concentration of 1 µg/ml and 300 U/ml of rhIL-2 into the T cell suspension to mix them well, seeding the cells in the coated 24-well plate, adding a corresponding concentration of mannose, blowing the mannose to homogeneity and culturing the cells in an incubator.
5. Infecting PBMCs with 1G4 TCR by means of viral centrifugation infection after culturing T cells for 48 hours.
6. After the viral infection of T cells, adding a corresponding concentration of mannose solution for further culture. Supplementing an equal amount of culture medium containing rhIL-2 and mannose every 2 days according to the growth status of T cells to maintain the density of T cells within a range of 0.5-2×10⁶/ml. On day 12 of the culture, detecting the expression level of transcription factors TCF-1 that regulate the differentiation of stem memory T cells in the culture by flow cytometry.
7. It was found by flow cytometry that on day 12 of the culture, the lymphocyte population primarily composed of stem memory T cells can be obtained. The results were shown in FIG. 4, compared with the control group, treatment with mannose significantly increased the expression of transcription factors TCF-1 that regulate the differentiation of stem memory T cells.

### Embodiment 4: Long-term in vitro expansion method using human-derived stem memory T cells as primary cell population

This embodiment includes the following steps:
1. Coating a cell culture plate with human CD3 antibody at a final concentration of 1 µg/ml.
2. Collecting 10 ml of peripheral blood from healthy people in a centrifuge tube, diluting the peripheral blood with phosphate buffer solution (PBS) at a ratio of 1:1, and mixing them evenly; adding 20 mL of lymphocyte separation medium (ficoll) into another new 50 mL centrifuge tube, then mixing the ficoll and blood dilution in a volume ratio of 1:1 to obtain the mixed blood dilution, slowly adding the mixed blood dilution along the tube wall to the upper layer of the lymphocyte separation medium to form a clear stratification between them, and centrifuging them at 800g for 20 min with an acceleration of 0 and a deceleration of 0.
3. At the end of the centrifugating operation, pipetting cells from the monocyte layer into a 50 mL centrifuge tube, washing the cells once by adding 20 mL of PBS, centrifuging them at 800 g for 5 min, discarding the supernatant; then resuspending with T cell culture medium and counting the cells, and diluting the cells to 1 million/ml to obtain T cell suspension.
4. Then adding human CD28 antibody at a final concentration of 1 µg/ml and 300 U/ml of rhIL-2 into the T cell suspension to mix well, seeding the cells in the coated 24-well plate, adding a corresponding concentration of mannose, blowing the mannose to homogeneity and culturing the cells in an incubator.
5. Supplementing an equal amount of culture medium containing rhIL-2 and mannose every 2 days according to the growth status of T cells to maintain the density of T cells within a range of 0.5-2×10⁶/ml. On day 12 of the culture (CE12& ME12), detecting the proportion of stem memory T cells and the expression level of transcription factors TCF-1 that regulate the differentiation of stem memory T cells in the culture by flow cytometry.
6. To further investigate the effects of mannose on the long-term expansion of stem memory T cells and the maintenance of stemness, continually stimulating the T cells that have been cultured to day 12 with human CD3 antibody at a final concentration of 1 µg/ml for 24 hours. On the next day, transferring the T cells to a new cell culture plate for further culturing the T cells to day 24. After then, continually stimulating the T cells that have been cultured to day 24 with human CD3 antibody at a final concentration of 1 µg/ml for 24 hours, then transferring the T cells to a new cell culture plate for further culturing the T cells to day 36 (CE36&ME36). Detecting the proportion of stem memory T cells and the expression level of transcription factors TCF-1 that regulate the differentiation of T cells in the culture by flow cytometry.
7. It was found by flow cytometry that, on days 12 and 36 of the culture, the lymphocyte population primarily composed of stem memory T cells can be obtained. The results were shown in FIG. 5: the proportion of CD45RO⁺CD27⁺ stem memory T cells in the mannose treatment group was significantly higher than that of the control group under an ordinary culture condition, meanwhile the expression of transcription factors TCF-1 that regulate the stemness of T cells in the mannose-treated T cells was also significantly higher than that of the control group.

### Embodiment 5: Long-term in vitro expansion method of 1G4 TCR T cells using stem memory T cells as primary cell population

This embodiment includes the following steps:
1. Coating a cell culture plate with human CD3 antibody at a final concentration of 1 µg/ml.
2. Collecting 10 ml of peripheral blood from healthy people in a centrifuge tube, diluting the peripheral blood with phosphate buffer solution (PBS) at a ratio of 1:1, and mixing them evenly; adding 20 mL of lymphocyte separation medium (ficoll) into another new 50 mL centrifuge tube, then mixing the ficoll and blood dilution in a volume ratio of 1:1 to obtain the mixed blood dilution, slowly adding the mixed blood dilution along the tube wall to the upper layer of the lymphocyte separation medium to form a clear stratification between them, and centrifuging them at 800g for 20 min with an acceleration of 0 and a deceleration of 0.
3. At the end of the centrifugating operation, pipetting cells from the monocyte layer into a 50 mL centrifuge tube, washing the cells once by adding 20 mL of PBS, centrifuging them at 800 g for 5 min, discarding the supernatant; then resuspending with T cell culture medium and counting the cells, and diluting the cells to 1 million/ml to obtain T cell suspension.
4. Then adding human CD28 antibody at a final concentration of 1 µg/ml and 300 U/ml of rhIL-2 into the T cell suspension to mix them well, seeding the cells in the coated 24-well plate, adding a corresponding concentration of mannose, blowing the mannose to homogeneity and culturing the cells in an incubator.
5. Infecting PBMCs with 1G4 TCR by means of viral centrifugation infection after culturing T cells for 48 hours.
6. After the viral infection of T cells, adding a corresponding concentration of mannose solution for further culture. Supplementing an equal amount of culture medium containing rhIL-2 and mannose every 2 days according to the growth status of T cells to maintain the density of T cells within a range of 0.5-2× 10⁶/ml. On day 12 of the culture (CE12& ME12), detecting the expression level of transcription factors TCF-1 that regulate the differentiation of stem memory T cells in the culture by flow cytometry.
7. To further investigate the effects of mannose on the long-term expansion of stem memory T cells and the maintenance of stemness, continually stimulating the T cells that have been cultured to day 12 with human CD3 antibody at a final concentration of 1 µg/ml for 24 hours. On the next day, transferring the T cells to a new cell culture plate for further culturing the T cells to day 24. After then, continually stimulating the T cells that have been cultured to day 24 with human CD3 antibody at a final concentration of 1 µg/ml for 24 hours, then transferring the T cells to a new cell culture plate for further culturing the T cells to day 36 (CE36&ME36). Detecting the proportion of stem memory T cells and the expression level of transcription factors TCF-1 that regulate the differentiation of T cells in the culture by flow cytometry.
8. It was found by flow cytometry that on days 12 and 36 of the culture, the lymphocyte population primarily composed of stem memory T cells can be obtained. The results were shown in FIG. 6, compared with the control group, the treatment with mannose significantly increased the expression of transcription factors TCF-1 that regulate the differentiation of stem memory T cells.

### Embodiment 6: Long-term in vitro expansion method of TIL cells using stem memory T cells as primary cell population

This embodiment includes the following steps:
1. Coating a cell culture plate with human CD3 antibody at a final concentration of 1 µg/ml.
2. Collecting 10 ml of peripheral blood from patients with liver cancer in a centrifuge tube, diluting the peripheral blood with phosphate buffer solution (PBS) at a ratio of 1:1, and mixing them evenly; adding 20 mL of lymphocyte separation medium (ficoll) into another new 50 mL centrifuge tube, then mixing the ficoll and blood dilution in a volume ratio of 1:1 to obtain the mixed blood dilution, slowly adding the mixed blood dilution along the tube wall to the upper layer of the lymphocyte separation medium to form a clear stratification between them, and centrifuging them at 800g for 20 min with an acceleration of 0 and a deceleration of 0.
3. At the end of the centrifugating operation, pipetting cells from the monocyte layer into a 50 mL centrifuge tube, washing the cells once by adding 20 mL of PBS, centrifuging them at 800 g for 5 min, discarding the supernatant; then resuspending with T cell culture medium and counting the cells, and diluting the cells to 1 million/ml to obtain T cell suspension.
4. Then adding human CD28 antibody at a final concentration of 1 µg/ml and 300 U/ml of rhIL-2 into the T cell suspension to mix them well, seeding the cells in the coated 24-well plate, adding a corresponding concentration of mannose, blowing the mannose to homogeneity and culturing the cells in an incubator.
5. After 48 hours of the T cells culture, supplementing an equal amount of culture medium containing rhIL-2 and mannose every 2 days according to the growth status of T cells to maintain the density of T cells within a range of 0.5-2×10⁶/ml. On day 12 of the culture (CE12& ME12), detecting the expression level of transcription factors TCF-1 that regulate the differentiation of stem memory T cells in the culture by flow cytometry.
6. To further investigate the effects of mannose on the long-term expansion of stem memory T cells and the maintenance of stemness, continually stimulating the T cells that have been cultured to day 12 with human CD3 antibody at a final concentration of 1 µg/ml for 24 hours. On the next day, transferring the T cells to a new cell culture plate for further culturing the T cells to day 24. After then, continually stimulating the T cells that have been cultured to day 24 with human CD3 antibody at a final concentration of 1 µg/ml for 24 hours, then transferring the T cells to a new cell culture plate for further culturing the T cells to day 36 (CE36&ME36). Detecting the proportion of stem memory T cells and the expression level of transcription factors TCF-1 that regulate the differentiation of T cells in the culture by flow cytometry.
7. It was found by flow cytometry that on days 12 and 36 of the culture, the lymphocyte population primarily composed of stem memory T cells can be obtained. The results were shown in FIG. 7, compared with the control group, the long-term mannose exposure significantly increased the expression of transcription factors TCF-1 that regulate the differentiation of stem memory T cells.

### Embodiment 7 Detection of in vivo anti-tumor activity of mannose-induced mouse stem memory T cells

This embodiment includes the following steps:
1. The *in vivo* anti-tumor activity of mannose-induced stem memory T cells in mice was carried out in 4-8-week-old C57BL6/N mice. In order to demonstrate the stem memory T cells obtained from the mannose treatment have better anti-tumor function and persistence compared with normally treated T cells, the present disclosure intended to use the OT-I T cell model to explore the *in vivo* anti-tumor activity of mannose-induced stem memory T cells.
2. Firstly, C57BL6/N mice were subcutaneously inoculated with 2×10⁵ B16-OVA cells at the axilla of the right hind limb. When the tumor cells had grown subcutaneously in the mice for about 8-10 days, the tumor volume was measured using a caliper. When the tumor volume reached 75mm³ to 150mm³, the C57BL6/N tumor-bearing mice were intravenously injected with 4×10⁶ mannose-treated or untreated OT-I T cells through the tail vein. Subsequently, the tumor volume was measured every 2 days, and the spleens and tumor tissues were obtained from the C57BL6/N mice on about day 14 (the specific operation time was depended on the experimental phenomenon). The tumor tissues from the mice were grounded and then subjected to Percoll density gradient centrifugation, and analyzed after flow cytometry staining.
3. The results were shown in FIG. 8. Firstly, it was found that, compared with the control group, the tumor growth rate of mice in the experimental group of mannose-treated adoptive T cells was significantly slowed down. Additionally, it can be seen from the results of flow cytometry staining that the percentage of infiltration of OT-I T cells treated with mannose in tumor tissues of tumor-bearing mice increased from about 1.0% to about 10%; meanwhile, the proportion of infiltrating T cells in the spleen was also significantly increased, indicating that mannose-treated OT-I T cells exhibited stronger anti-tumor effect and persistence in adoptive mouse models.

### Embodiment 8 Detection of in vivo anti-tumor activity of human stem memory T cells induced by long-term mannose treatment

This embodiment includes the following steps:
1. The *in vivo* anti-tumor activity of mannose-induced stem memory T cells in mice was carried out in 8-10-week-old NSG mice. In order to demonstrate the stem memory T cells obtained from mannose treatment have better anti-tumor activity and persistence compared with normally treated T cells, the present disclosure intended to use the 1G4 TCR T cell model to explore the *in vivo* anti-tumor activity of stem memory T cells treated with mannose *in vitro* for a long term.
2. Firstly, NSG mice were subcutaneously inoculated with 3×10⁶ HepG2-NYESO tumor cells at the axilla of the right hind limb. When the tumor cells had grown subcutaneously in the mice for about 8-10 days, the tumor volume was measured using a caliper. When the tumor volume reached 75 mm³ to 150 mm³, the NSG tumor-bearing mice were intravenously injected with 6×10⁶ mannose-treated or untreated 1G4 TCR T(CE12/ME12/CE36/ME36) cells through the tail vein. Subsequently, the tumor volume was measured every 2 days, and the tumor tissues were obtained from the NSG mice on about day 14 (the specific operation time was depended on the experimental phenomenon). The tumor tissues from the mice were grounded and then subjected to Percoll density gradient centrifugation, and analyzed after flow cytometry staining.
3. The results were shown in FIG. 9. Firstly, it was found that, compared with the control group, the tumor growth rate of mice in the experimental group of adoptive T cells treated with mannose for a long term was significantly slowed down, indicating that mannose-treated 1G4 TCR T cells exhibited stronger anti-tumor effect in adoptive mouse models.

It should be understood that the above embodiments are exemplary and are not intended to encompass all possible implementations included in the claims. Various modifications and changes can also be made on the basis of the above embodiments without departing from the scope of the present disclosure. Likewise, the various technical features of the above embodiments may be arbitrarily combined to form additional embodiments of the present disclosure that may not have been explicitly described. Therefore, the above embodiments only illustrate several implementations of the present disclosure and do not limit the scope of protection of the present disclosure.

## Claims

1. A method for inducing and expanding a stem memory T cell *in vitro,* wherein the method comprises the following steps:
S1: activating and expanding a T cell;
S2: adding mannose to a culture medium to culture the T cell during or after the activation of the T cell.

2. The method according to claim 1, wherein the step S1 comprises: coating a cell culture plate with an antibody, then activating and expanding the T cell in a culture medium containing a cytokine;
preferably, the antibody is a CD3 antibody and a CD28 antibody;
preferably, the cytokine is selected from one or more of a group consisting of IL-2, IL-7, IL-15, IL-17, and IL-21, and preferably IL-2.

3. The method according to claim 2, wherein a concentration of the CD3 antibody is 1-2 µg/mL;
preferably, a concentration of the CD28 antibody is 1-2 µg/mL.

4. The method according to any one of claims 1-2, wherein a concentration of the mannose is 2.0-100.0 mM; and preferably 5.0-50.0 mM.

5. The method according to any one of claims 1-4, wherein the method further comprises the following step:
S3: replacing the culture medium with a culture medium containing a cytokine and mannose for culture to obtain a lymphocyte population primarily composed of the stem memory T cell;
preferably, the cytokine is selected from one or more of a group consisting of IL-2, IL-7, IL-15, IL-17, and IL-21, and preferably IL-2;
preferably, time for replacing the culture medium is 1 to 3 days, preferably 2 days;
preferably, the lymphocyte population comprises CD4 T cells, CD8 T cells, γδ T, TILs, TCR-T, CAR-T, and STAR-T.

6. The method according to any one of claims 1-4, wherein the method further comprises the following step:
S4: stimulating the T cell obtained in the step S3 with an antibody for further culture;
preferably, the culture is conducted for 12 days;
preferably, the step S4 is conducted cyclically for two or more times;
preferably, the antibody is a CD3 antibody and/or a CD28 antibody, preferably the CD3 antibody;
preferably, a concentration of the antibody is 0.5-2 µg/mL, preferably 1 µg/mL.

7. The method according to any one of claims 1-6, wherein the T cell comprises a human-derived T cell or a murine-derived T cell.

8. A lymphocyte population, wherein the lymphocyte population is prepared by the method according to any one of claims 1-7.

9. A pharmaceutical composition, wherein the pharmaceutical composition comprises the lymphocyte population according to claim 8;
preferably, the pharmaceutical composition further comprises a pharmaceutically acceptable excipient;
preferably, the pharmaceutical composition further comprises an additional therapeutic agent.

10. A use of the lymphocyte population prepared by the method according to any one of claims 1-7, the lymphocyte population according to claim 8 or the pharmaceutical composition according to claim 9 in the preparation of a drug for adoptive immunotherapy;
preferably, the adoptive immunotherapy is anti-tumor immunotherapy, anti-infection immunotherapy, and autoimmune disease treatment.

11. A use of mannose in inducing and expanding a stem memory T cell *in vitro.*
